# FASCICULE DE BREVET EUROPEEN

(11) **EP 1 528 902 B1**
(45) Date de publication et mention de la délivrance du brevet: **15.11.2006**
(21) Numéro de dépôt: 02767735.0
(22) Date de dépôt: 15.08.2002
(51) Int. Cl.: A61F 2/38

(54) **PROTHESE DE GENOU**
KNIEPROTHESE
KNEE PROSTHESIS

(43) Date de publication de la demande: 11.05.2005
(73) Titulaire: Symbios Orthopédie SA, 1400 Yverdon-les-Bains (CH)
(72) Inventeur: LEYVRAZ, Pierre-François, 1806 St-Légier (CH)
(74) Mandataire: Micheli & Cie SA
(86) Numéro de dépôt international: PCT/IB2002/003276
(87) Numéro de publication internationale: WO 2004/016204

(56) Documents cités:
- EP-A- 0 993 812
- WO-A-96/20656
- FR-A- 2 619 306

## Description

La présente invention se rapporte au domaine des prothèses médicales du genou.

On connaît par le document WO 96/20656 une prothèse de genou constituée d'un implant fémoral et d'un implant tibial. L'implant tibial comprend une embase tibiale et un insert tibial monté mobile en rotation sur l'embase tibiale. L'implant fémoral comprend deux condyles et un rebord antérieur définissant ensemble une surface de contact avec le fémur et une surface d'articulation convexe. La surface de contact avec le fémur comprend deux surfaces planes postérieures, deux chanfreins plans postérieurs, deux surfaces planes distales, un chanfrein plan antérieur et une surface plane antérieure. La surface d'articulation est constituée par les surfaces articulaires respectives des condyles et la surface articulaire du rebord antérieur.

Selon une caractéristique particulière de cette prothèse, le profil sagittal de l'implant fémoral présente, quelle que soit la taille de l'implant fémoral, une géométrie et des dimensions constantes, sauf dans sa partie antérieure. Plus précisément, la longueur et l'orientation, dans le plan sagittal, des surfaces planes postérieures, des chanfreins plans postérieurs et des surfaces planes distales de la surface de contact avec le fémur sont constantes quelle que soit la taille de l'implant fémoral, de même que le profil des surfaces articulaires des condyles. Seules varient dans le plan sagittal en fonction de la taille de l'implant fémoral la longueur du chanfrein antérieur et le profil de la surface articulaire du rebord antérieur.

Le fait de conserver une géométrie et des dimensions sagittales constantes dans les parties de l'implant fémoral autres que la partie antérieure permet de réduire le nombre d'instruments de coupe nécessaires pour la préparation du fémur lors de l'intervention chirurgicale.

Toutefois, cet avantage a pour contrepartie une dégradation de la conformité géométrique de la prothèse à l'anatomie naturelle du genou. Un implant fémoral de grande taille aura en effet, dans le plan sagittal, la même surface d'appui sur l'insert tibial qu'un implant fémoral de petite taille, contrairement à l'anatomie du genou. En outre, les implants fémoraux de grande taille présentent un risque de subluxation important du fait que, nécessairement, leur rebord antérieur déborde largement par rapport à la surface d'appui de l'insert tibial, ce qui déséquilibre la géométrie d'ensemble de la prothèse. En raison de ces inconvénients, il ne paraît pas possible, avec la prothèse selon le document WO 96/20656, de couvrir toutes les tailles fémorales existantes.

Le document FR 2 619 306 décrit un implant fémoral dont la surface d'articulation est différente en fonction de la taille. Cet implant ne permet pas de réduire le nombre d'instruments de coupe nécessaires pour la préparation du fémur comme dans le document WO 96/20656.

On connaît également des prothèses de genou, dites postéro-stabilisées, dans lesquelles l'implant fémoral comprend une came. Cette came est généralement disposée en partie postérieure supérieure de l'implant fémoral, dans une échancrure intercondylienne séparant deux condyles de l'implant fémoral. Un plot central saillant sur la face supérieure de l'implant tibial vient se placer dans l'échancrure intercondylienne pour coopérer avec la came à partir d'un certain angle de flexion du genou de façon à empêcher l'implant fémoral de se déplacer antérieurement par rapport à l'implant tibial à partir de cet angle de flexion,

Dans ces prothèses postéro-stabilisées, la surface articulaire, ou surface d'appui, de chaque condyle a un grand rayon de courbure en partie distale et de plus petits rayons de courbure en partie postérieure pour augmenter le degré de flexion. Généralement, cette surface articulaire est en outre congruente avec la surface articulaire correspondante de l'implant tibial en partie distale, jusqu'à un angle de flexion du genou inférieur à l'angle de flexion précité auquel la came entre en contact avec le plot. En d'autres termes, la surface articulaire de chaque condyle a, dans le plan sagittal, et jusqu'à cet angle de flexion inférieur, un profil en arc de cercle de rayon sensiblement égal à celui du profil, également en arc de cercle, de la surface articulaire correspondante de l'implant tibial. Ainsi, le mouvement de flexion du genou comporte trois phases :
- une première phase, dite de glissement, allant d'un angle de flexion de 0° (position d'extension du genou) à l'angle de flexion auquel la congruence dans le plan sagittal entre la surface articulaire de chaque condyle et la surface articulaire correspondante de l'implant tibial se termine, au cours de laquelle l'implant fémoral pivote par rapport à l'implant tibial autour d'un axe constant et les surfaces articulaires de l'implant fémoral glissent sur celles de l'implant tibial ;
- une seconde phase, dite phase intermédiaire, allant de l'angle de flexion auquel la congruence se termine à l'angle de flexion auquel la came entre en contact avec le plot, au cours de laquelle l'implant fémoral pivote autour d'un axe et peut se déplacer en translation dans la direction antéro-postérieure par rapport à l'implant tibial ; et
- une troisième phase, dite de roulement, allant de l'angle de flexion où la came entre en contact avec le plot jusqu'à la fin de la flexion, au cours de laquelle l'implant fémoral roule postérieurement sur l'implant tibial.

Durant la phase intermédiaire, les frottements entre les implants fémoral et tibial peuvent être très importants, causant une usure des surfaces articulaires, généralement en polyéthylène, de l'implant tibial. De plus, l'absence de congruence entre les surfaces articulaires des implants fémoral et tibial fait que la came entre en contact brutalement avec le plot, avec comme conséquence un risque de fracture du plot. Comme il n'est pas stabilisé dans la direction antéro-postérieure pendant cette phase, l'implant fémoral a en outre tendance à se déplacer antérieurement, ce qui, d'une part, augmente les contraintes au niveau de l'interface entre le rebord antérieur de l'implant fémoral et la rotule et, d'autre part, diminue le bras de levier et rend ainsi le fléchissement du genou plus difficile pour le patient.

La présente invention vise, en premier lieu, à proposer un système prothétique pour le genou qui permette, comme la prothèse selon le document WO 96/20656, de réduire le nombre d'instruments de coupe nécessaires à la préparation du fémur, mais qui respecte mieux l'anatomie naturelle du genou.

A cette fin, il est prévu, selon un premier aspect de l'invention, un système prothétique pour le genou comprenant un ensemble d'implants fémoraux de tailles respectives croissantes, chacun desdits implants fémoraux comprenant au moins un condyle et un rebord antérieur, le ou chaque condyle comprenant des surfaces planes postérieure et distale de contact avec le fémur et une surface articulaire, le rebord antérieur comprenant un chanfrein plan antérieur de contact avec le fémur, l'orientation dans le plan sagittal de la ou chaque surface plane postérieure, l'orientation et la longueur dans le plan sagittal de la ou chaque surface plane distale et l'orientation dans le plan sagittal du chanfrein plan antérieur étant identiques pour tous les implants fémoraux dudit ensemble, la longueur dans le plan sagittal du chanfrein antérieur augmentant avec la taille pour tous les implants fémoraux dudit ensemble, caractérisé en ce que le rayon de courbure dans le plan sagittal d'une partie distale de la surface articulaire du ou de chaque condyle augmente avec la taille pour au moins deux implants fémoraux dudit ensemble.

Ainsi, dans ce premier aspect de l'invention, l'on dissocie l'évolution en fonction de la taille de la surface de contact avec le fémur et de la surface articulaire des condyles en partie distale de l'implant fémoral. Comme le rayon de la partie distale de la surface articulaire de chaque condyle augmente avec la taille pour au moins deux implants fémoraux de l'ensemble, l'anatomie du genou est mieux respectée. L'on peut dès lors couvrir une plus grande plage de tailles fémorales.

Avantageusement, la distance entre un plan coronal de référence tangent à la surface articulaire du ou de chaque condyle lorsque le genou est en extension et le point le plus distal du profil sagittal de la surface articulaire du ou de chaque condyle augmente aussi avec la taille pour lesdits au moins deux implants fémoraux.

De préférence, le rayon de courbure dans le plan coronal de la partie distale de la surface articulaire du ou de chaque condyle augmente aussi avec la taille pour lesdits au moins deux implants fémoraux.

De préférence, également, la longueur dans le plan sagittal de la surface plane postérieure de contact avec le fémur du ou de chaque condyle augmente avec la taille pour tous les implants fémoraux dudit ensemble.

Le ou chaque condyle de chaque implant fémoral peut comprendre en outre, entre sa surface plane postérieure et sa surface plane distale, un chanfrein plan postérieur de contact avec le fémur, l'orientation et la longueur dans le plan sagittal de ce chanfrein plan postérieur étant identiques pour tous les implants fémoraux dudit ensemble.

Le rebord antérieur de chaque implant fémoral peut comporter en outre une surface articulaire et, entre le chanfrein plan antérieur et une extrémité supérieure du rebord antérieur, une surface plane antérieure de contact avec le fémur. Dans ce cas, la surface articulaire du rebord antérieur peut présenter une gorge destinée à coopérer avec la rotule ou un implant rotulien, et une surépaisseur ayant un profil sagittal triangulaire peut être prévue sur la surface de contact avec le fémur du rebord antérieur au niveau de l'angle formé par le chanfrein plan antérieur et la surface plane antérieure et dans une zone opposée à la gorge, cette surépaisseur définissant un chanfrein dont le profil sagittal a un point d'origine sur le chanfrein plan antérieur et une orientation qui sont identiques pour tous les implants fémoraux dudit ensemble et une longueur qui augmente avec la taille pour tous les implants fémoraux dudit ensemble.

Avantageusement, l'épaisseur d'une partie distale du ou de chaque condyle et l'épaisseur d'une partie postérieure du ou de chaque condyle sont identiques pour tous les implants fémoraux dudit ensemble, et, pour chaque implant fémoral, l'épaisseur de la partie distale du ou de chaque condyle est sensiblement égale à l'épaisseur de la partie postérieure du ou de chaque condyle.

Dans un mode de réalisation typique de l'invention, chaque implant fémoral comprend deux condyles, la partie distale de la surface articulaire de chaque condyle est sensiblement une portion de sphère, et l'écartement entre les centres de courbure respectifs des parties distales des surfaces articulaires des condyles augmente avec la taille pour lesdits au moins deux implants fémoraux. Le rayon de courbure de la partie distale de la surface articulaire de chaque condyle ainsi que l'écartement entre les centres de courbure respectifs des parties distales des surfaces articulaires des condyles peuvent néanmoins être identiques pour au moins deux implants fémoraux dudit ensemble. Le rayon de courbure et l'écartement précités peuvent ainsi, par exemple, augmenter toutes les deux tailles d'implant fémoral.

Le système prothétique selon l'invention peut comprendre en outre au moins un implant tibial, de préférence un ensemble d'implants tibiaux de tailles différentes, le ou chaque implant tibial comprenant au moins une surface articulaire apte(s) à coopérer avec la ou les surfaces articulaires du ou des condyles d'au moins un implant fémoral correspondant pour permettre une flexion du genou. De préférence, la ou chaque surface articulaire du ou de chaque implant tibial est sensiblement une portion de sphère de même rayon que le rayon de la partie distale de la surface articulaire du ou de chaque condyle du ou de chaque implant fémoral correspondant.

La présente invention vise, en second lieu, à remédier aux inconvénients mentionnés ci-dessus relatifs aux prothèses de genou postéro-stabilisées.

A cette fin, il est prévu, selon un second aspect de l'invention, une prothèse de genou postéro-stabilisée comprenant un implant fémoral et un implant tibial, l'implant fémoral comprenant au moins une surface articulaire condylienne et une came, l'implant tibial comprenant au moins une surface articulaire destinée(s) à coopérer avec la ou les surfaces articulaires condyliennes de l'implant fémoral pour permettre une flexion de la prothèse et un plot destiné à coopérer avec la came à partir d'un certain angle de flexion de la prothèse pour stabiliser postérieurement l'implant fémoral par rapport à l'implant tibial, le profil sagittal de la ou chaque surface articulaire condylienne étant congruent avec le profil sagittal de la surface articulaire correspondante de l'implant tibial pour une flexion de la prothèse allant au moins jusqu'à un angle déterminé, caractérisée en ce que la came et le plot sont agencés pour que la came entre en contact avec le plot à un angle de flexion de la prothèse sensiblement égal à l'angle déterminé.

Ainsi, dans ce second aspect de l'invention, le contact entre la came et le plot intervient alors que les surfaces articulaires condyliennes sont congruentes avec les surfaces articulaires correspondantes de l'implant tibial. L'implant fémoral passe ainsi directement d'une phase de glissement à une phase de roulement. Tous les inconvénients des prothèses actuelles liées à l'existence d'une phase intermédiaire entre la phase de glissement et la phase de roulement sont donc supprimés.

Typiquement, la surface articulaire du plot est une surface plane postérieure qui est alignée avec le point le plus distal du profil sagittal de la ou chaque surface articulaire condylienne lorsque le genou est en extension.

Le profil sagittal de la ou chaque surface articulaire condylienne comprend une portion sensiblement en arc de cercle s'étendant au moins depuis sensiblement le point le plus distal du profil sagittal et au moins jusqu'à sensiblement un point du profil sagittal situé postérieurement par rapport au point le plus distal et à une position telle que la droite passant par ce point postérieur et le centre de courbure de la portion en arc de cercle fasse l'angle déterminé avec la droite passant par le point le plus distal et le centre de courbure. Sensiblement à partir du point postérieur, et jusqu'à une extrémité postérieure supérieure du profil sagittal, ledit profil sagittal est de préférence sensiblement un arc de cercle de rayon inférieur au rayon de la portion en arc de cercle.

D'autres caractéristiques et avantages de la présente invention apparaîtront à la lecture de la description détaillée suivante de plusieurs modes de réalisation faite en référence aux dessins annexés dans lesquels :
- la figure 1 est une vue sagittale (vue de profil) d'une prothèse de genou selon l'invention ;
- la figure 2 est une vue coronale (vue frontale) de la prothèse de genou selon l'invention ;
- la figure 3 est une vue en perspective d'un implant fémoral faisant partie de la prothèse de genou selon l'invention ;
- la figure 4 est une vue sagittale d'une embase tibiale d'un implant tibial faisant partie de la prothèse de genou selon l'invention ;
- la figure 5 est une vue en perspective d'un insert tibial d'un implant tibial faisant partie de la prothèse de genou selon l'invention ;
- la figure 6 est une vue sagittale de l'insert tibial illustré à la figure 5 ;
- les figures 7A et 7B représentent en vue sagittale l'implant fémoral de la prothèse de genou selon l'invention ;
- les figures 8A à 8D sont des vues en coupe sagittale montrant quatre positions de flexion différentes de la prothèse de genou selon l'invention ;
- les figures 9 et 10 sont des vues schématiques, respectivement sagittale et coronale, de la prothèse de genou selon l'invention, représentant quatre tailles différentes d'implant fémoral et deux tailles différentes d'implant tibial, avec l'implant tibial coupé au niveau de sa partie supérieure pour mettre en évidence la partie inférieure de l'implant fémoral ; et
- les figures 11 et 12 sont respectivement des vues schématiques en coupe et de dessus d'un guide de coupe pouvant être utilisé pour préparer le fémur avant la mise en place de la prothèse de genou selon l'invention.

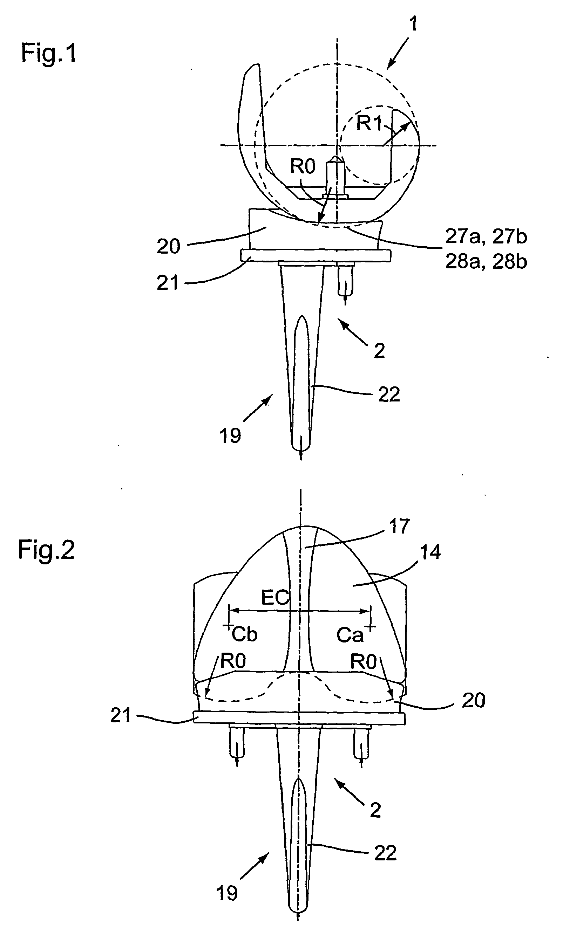

En référence aux figures 1 à 3, une prothèse de genou bi-condylienne postéro-stabilisée selon l'invention comprend un implant fémoral 1 et un implant tibial 2 destinés à être fixés respectivement au fémur et au tibia. L'implant rotulien de la prothèse n'est pas représenté car semblable à ceux existants.

L'implant fémoral 1 comprend, de part et d'autre d'une échancrure intercondylienne 3, deux condyles 4a, 4b réunis par un rebord antérieur 5, appelé également trochlée. Chaque condyle 4a, 4b est constitué d'une partie postérieure 6a, 6b et d'une partie distale 7a, 7b.

Le rebord antérieur 5 et les condyles 4a, 4b définissent ensemble une surface de contact avec le fémur et une surface d'articulation. La surface de contact avec le fémur comprend deux surfaces planes postérieures 8a, 8b, deux chanfreins plans postérieurs 9a, 9b, deux surfaces planes distales 10a, 10b, un chanfrein plan antérieur 11 et une surface plane antérieure 12. La surface d'articulation est convexe. Elle comprend les surfaces articulaires respectives, 13a, 13b, des condyles et la surface articulaire, 14, du rebord antérieur.

Sur les deux surfaces planes distales 10a, 10b de la surface de contact avec le fémur sont prévus deux plots 15a, 15b destinés à être introduits dans des trous correspondants pratiqués dans l'extrémité distale du fémur, postérieurement à l'axe intramédullaire, pour stabiliser en rotation l'implant fémoral, c'est-à-dire l'empêcher de tourner par rapport au fémur. Une surépaisseur 16 bordant la partie distale de l'échancrure intercondylienne 3, sur la surface de contact avec le fémur, et destinée à être reçue dans un évidement correspondant pratiqué dans l'extrémité distale du fémur, permet d'apporter une stabilité en rotation supplémentaire à l'implant fémoral.

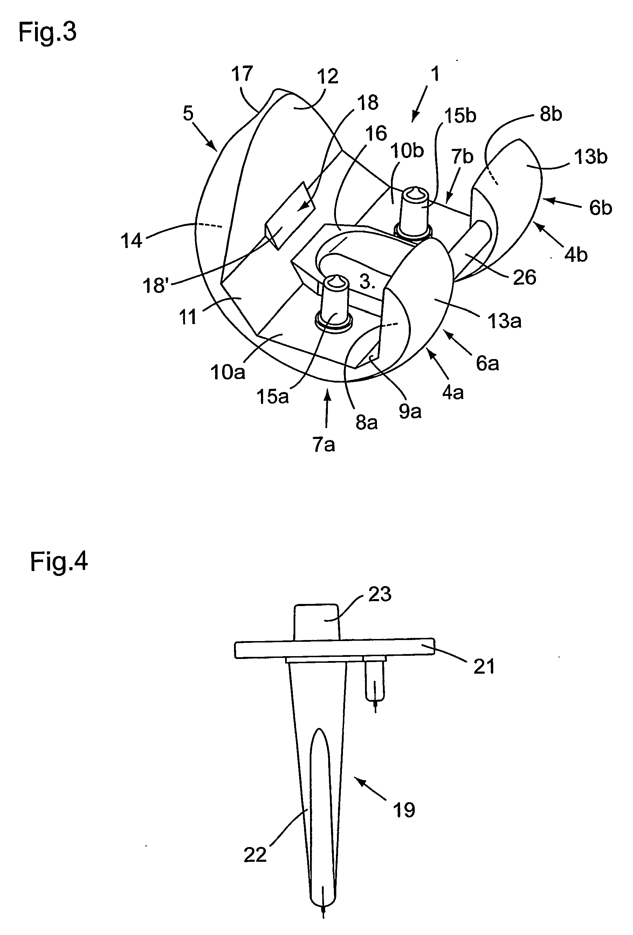

La surface d'articulation 14 du rebord antérieur présente, comme montré aux figures 2 et 3, une gorge 17 s'étendant centralement depuis l'extrémité supérieure de la surface d'articulation 14 jusqu'à l'échancrure intercondylienne 3. Cette gorge 17 a pour fonction de guider l'implant rotulien lors des mouvements du genou, d'une manière connue en soi. Afin de compenser le manque de matière dû à la gorge 17, une surépaisseur 18 est prévue sur la surface de contact avec le fémur dans une zone centrale opposée à la gorge 17. Cette surépaisseur 18 a un profil sagittal triangulaire et définit un chanfrein 18' au niveau de l'angle formé par le chanfrein antérieur 11 et la surface antérieure 12,

L'implant tibial 2 comprend une embase tibiale 19 et un insert tibial 20 monté mobile en rotation sur l'embase tibiale. L'embase tibiale 19 comprend un plateau 21 et une tige 22 d'ancrage dans le tibia, solidaire du plateau 21.
L'insert tibial 20, également appelé insert méniscal, présente sur sa face inférieure un orifice légèrement tronconique (non représenté) situé antérieurement dans le plan sagittal et centralement dans le plan coronal. Cet orifice tronconique reçoit un plot légèrement tronconique correspondant 23 saillant sur la face supérieure du plateau tibial 21 (figure 4), ce plot légèrement tronconique 23 autorisant un déplacement en rotation de l'insert tibial par rapport à l'embase tibiale.

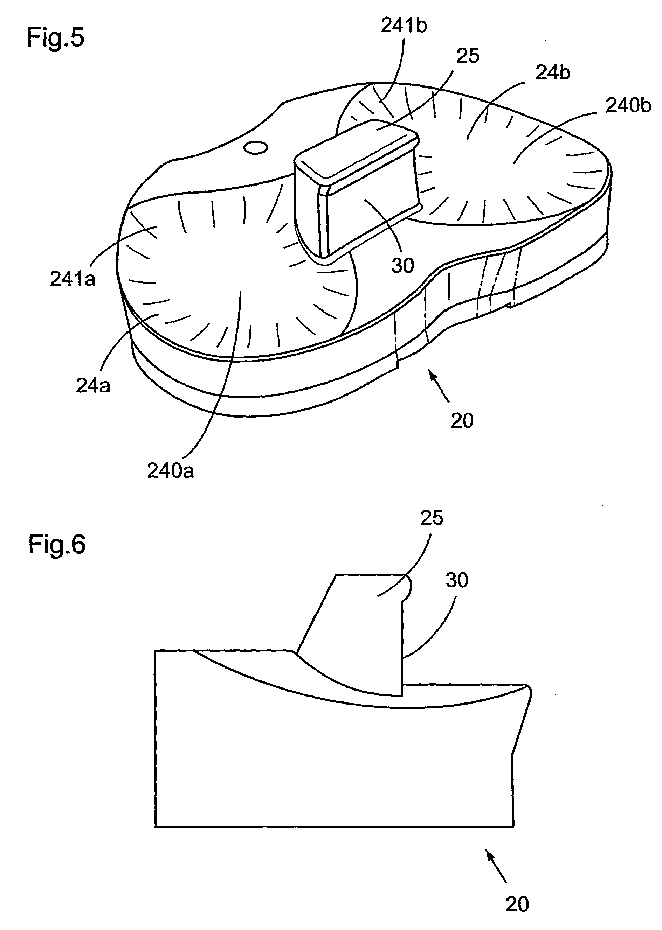

La face supérieure de l'insert tibial 20 présente deux dépressions 24a, 24b (figures 5, 6) dont les surfaces respectives constituent les surfaces articulaires de l'insert tibial destinées à coopérer avec les surfaces articulaires 13a, 13b des condyles et une partie inférieure de la surface articulaire 14 du rebord antérieur. Plus précisément, une portion principale 240a, 240b de la surface de chaque dépression 24a, 24b constitue une surface d'articulation pour la flexion de la prothèse, et une portion périphérique antérieure 241 a, 241 b ayant, dans le plan sagittal, un plus grand rayon de courbure que la portion principale 240a, 240b constitue une zone de dégagement contre laquelle peut venir s'appuyer la partie inférieure de la surface articulaire 14 du rebord antérieur lorsque le genou est en hyperextension.

Un plot 25, également appelé épine, est en outre saillant sur la face supérieure de l'insert tibial. Le plot 25 vient se placer dans l'échancrure intercondylienne 3 de l'implant fémoral et est adapté pour coopérer avec une came intercondylienne 26 prévue entre les condyles 4a, 4b, en partie postérieure de l'implant fémoral, pour postéro-stabiliser la prothèse, c'est-à-dire empêcher l'implant fémoral de se déplacer antérieurement par rapport à l'implant tibial lors de la flexion du genou, comme cela sera expliqué plus loin.

L'implant fémoral 1 est typiquement en métal, par exemple en cobalt-chrome, ou en céramique. L'embase tibiale 19 est typiquement en métal, par exemple en cobalt-chrome. L'insert tibial 20 est, lui, en polyéthylène et plus particulièrement en polyéthylène à ultra haut poids moléculaire.

Conformément à l'invention, les surfaces articulaires 13a, 13b des condyles et les surfaces articulaires 240a, 240b de l'insert tibial 20 sont conformées pour que chaque surface articulaire condylienne 13a, 13b soit congruente dans le plan sagittal avec la surface articulaire 240a, 240b correspondante pour une flexion du genou (de la prothèse) allant de 0° à 90°.

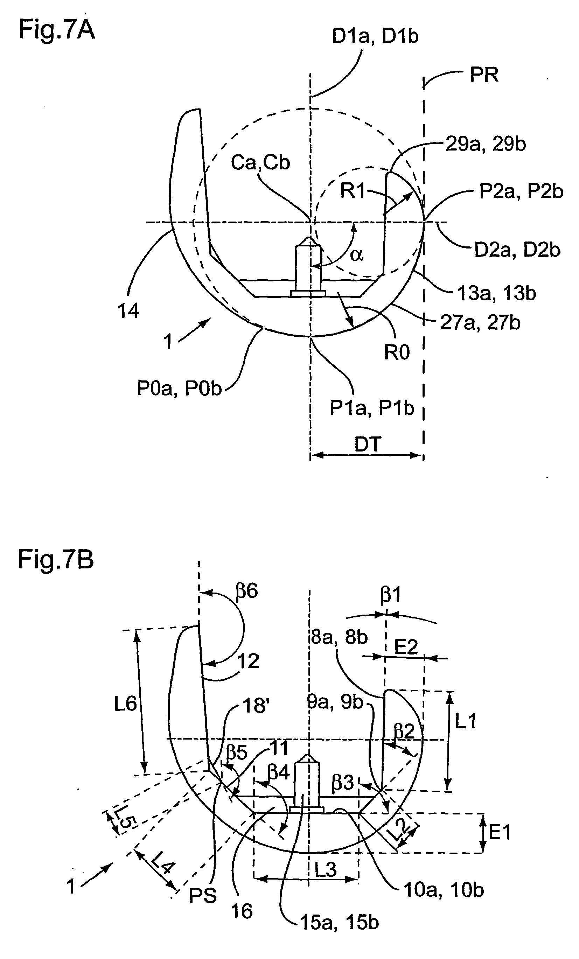

Ainsi, comme le montrent les figures 1 et 7A, le profil sagittal de la surface articulaire 13a, 13b de chaque condyle présente une portion en arc de cercle 27a, 27b s'étendant au moins depuis le point le plus distal, P1 a, P1 b, du profil sagittal, de préférence depuis un point P0a, P0b antérieur au point le plus distal P1 a, P1 b comme représenté à la figure 7A, et jusqu'à un point P2a, P2b du profil sagittal situé postérieurement par rapport au point le plus distal P1 a, P1 b et à une position telle que la droite, D2a, D2b, passant par ce point postérieur P2a, P2b et le centre de courbure, Ca, Cb, de la portion en arc de cercle 27a, 27b soit orthogonale à la droite, D1a, D1b, passant par le point le plus distal P1 a, P1 b et le centre de courbure Ca, Cb, et le profil sagittal, 28a, 28b, de chaque surface articulaire 240a, 240b de l'insert tibial est un arc de cercle sensiblement de même rayon que le rayon, R0, de la portion en arc de cercle 27a, 27b du profil sagittal de chaque surface articulaire condylienne 13a, 13b.

Le profil sagittal 28a, 28b de chaque surface articulaire de l'insert tibial 20 peut avoir exactement le même rayon R0 que la portion en arc de cercle 27a, 27b de chaque surface articulaire condylienne. Toutefois, de préférence, le rayon du profil sagittal 28a, 28b est très légèrement supérieur, d'une valeur maximale de 0,3 mm pour un rayon R0 de 22 mm à 30 mm, à celui de la portion en arc de cercle 27a, 27b, afin de réduire les contraintes exercées sur les surfaces articulaires 240a, 240b dues à la différence d'élasticité entre la matière dans laquelle est fait l'implant fémoral 1 et celle dans laquelle est fait l'insert tibial 20.

Cette forme des surfaces articulaires des condyles 4a, 4b et de l'insert tibial 20 permet à l'implant fémoral de glisser sur les surfaces articulaires de l'insert tibial 20, en pivotant autour de l'axe passant par les centres de courbures Ca, Cb, lors d'une flexion du genou allant de 0° à 90°. Durant cette phase de glissement, la prothèse est stable car la rotation de l'implant fémoral se fait autour d'un axe constant, à savoir l'axe précité passant par les centres de courbures Ca, Cb. En outre, le fait que les surfaces articulaires condyliennes 13a, 13b et les surfaces articulaires 240a, 240b de l'insert tibial 20 soient congruentes pour un si grand angle de flexion permet de répartir les contraintes de contact entre l'implant fémoral et l'implant tibial sur une grande surface de contact.

A partir du point postérieur P2a, P2b, et jusqu'à son extrémité postérieure supérieure, 29a, 29b, le profil sagittal de chaque surface articulaire condylienne 13a, 13b présente un rayon de courbure R1 inférieur au rayon R0 de la portion en arc de cercle 27a, 27b, ceci afin d'augmenter le degré de flexion de la prothèse. Ce rayon de courbure R1 est de préférence constant sur toute la portion du profil sagittal allant du point postérieur P2a, P2b à l'extrémité postérieure supérieure 29a, 29b, c'est-à-dire, en d'autres termes, que la portion du profil sagittal située entre les points P2a, P2b et 29a, 29b est de préférence un arc de cercle. Dans des exemples de réalisation typiques, le rayon de courbure R1 est choisi de façon que le degré de flexion associé à cette portion du profil sagittal se situe dans une plage allant de 65° à 75°, afin d'obtenir un degré de flexion total pour la prothèse de 155° à 165°.

De préférence, la partie de la surface articulaire 13a, 13b de chaque condyle ayant le profil sagittal en arc de cercle 27a, 27b est une portion de sphère, et a donc un rayon R0 identique dans le plan sagittal et le plan coronal, comme illustré aux figures 1 et 2, et les surfaces articulaires 240a, 240b de l'insert tibial sont également des portions de sphère, sensiblement de même rayon que le rayon R0. Les surfaces articulaires des condyles et de l'insert tibial sont ainsi congruentes également dans le plan coronal. Quant à la partie postérieure supérieure de la surface articulaire 13a, 13b de chaque condyle, c'est-à-dire la partie ayant le profil sagittal en arc de cercle de rayon R1, elle a de préférence un profil en arc de cercle de rayon R0 dans le plan coronal.

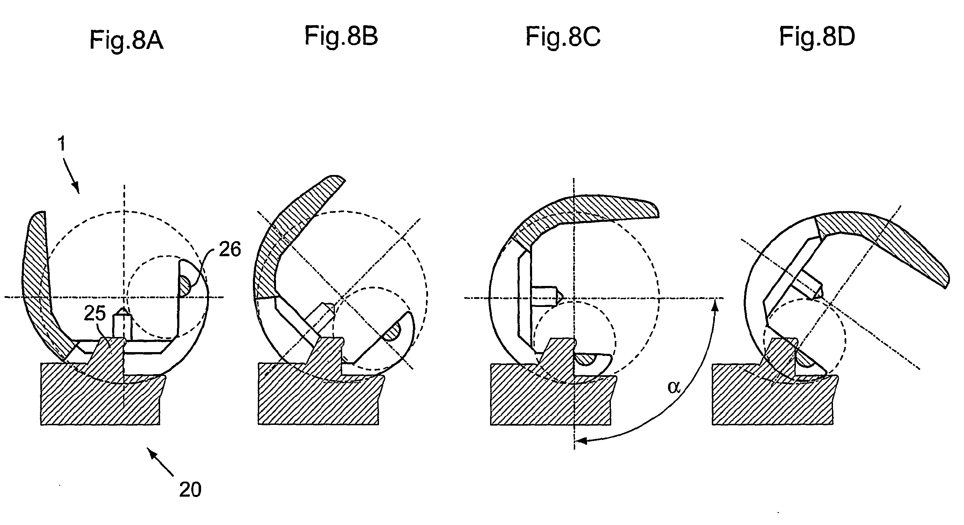

La came intercondylienne 26 est une barre en forme de demi-cylindre joignant les deux condyles 4a, 4b. La surface articulaire de la came 26 est constituée par la surface à profil sagittal semi-circulaire du demi-cylindre. La came 26 est tangente au plan contenant les droites D2a, D2b associées au profil sagittal de chaque condyle et est située par rapport à ce plan du côté de l'extrémité postérieure supérieure 29a, 29b des condyles (figures 7A, 8A).

Le plot 25 de l'insert tibial présente une surface postérieure plane 30 qui est alignée avec les points les plus distaux P1a, P1b des surfaces articulaires condyliennes 13a, 13b, c'est-à-dire située dans le même plan coronal que les points P1a, P1b, lorsque le genou est en extension. Cette surface postérieure plane 30 constitue la surface articulaire du plot 25, destinée à coopérer avec la surface articulaire de la came 26.

Les figures 8A à 8D montrent l'implant fémoral dans différentes positions de flexion. On notera que, du fait de leurs positions relatives, la came 26 et le plot 25 entrent en contact l'un avec l'autre à un angle de flexion α de 90° (figure 8C), soit juste à la transition entre le rayon de courbure R0 et le rayon de courbure R1 du profil sagittal de la surface articulaire de chaque condyle. L'implant fémoral passe ainsi directement d'une phase de glissement sur l'insert tibial à une phase de roulement. Le fait, selon l'invention, de supprimer la phase intermédiaire qui existe dans les prothèses de genou postéro-stabilisées actuelles entre la phase de glissement et la phase de roulement, présente notamment les avantages suivants :
- l'usure des surfaces articulaires de l'insert tibial 20 est réduite,
- le contact entre la came 26 et le plot 25 se fait de manière moins brutale,
- les contraintes exercées entre le rebord antérieur 5 de l'implant fémoral et l'implant rotulien (ou la rotule lorsque celle-ci n'est pas remplacée) sont moins élevées, et
- le bras de levier et plus important, ce qui facilite le fléchissement du genou pour le patient.

Par ailleurs, en ne faisant entrer en contact la came 26 et le plot 25 qu'à un angle de flexion de 90°, l'on réduit encore davantage les contraintes exercées sur le plot 25. C'est en effet entre 0° et 90° en flexion que les charges dans un genou sont les plus élevées (de telles charges peuvent aller jusqu'à huit fois le poids du corps selon les activités), le pic de force étant à 20° pour la marche, 50° pour la montée en pente, 60° pour la montée d'escalier et 90° pour la descente d'escalier.

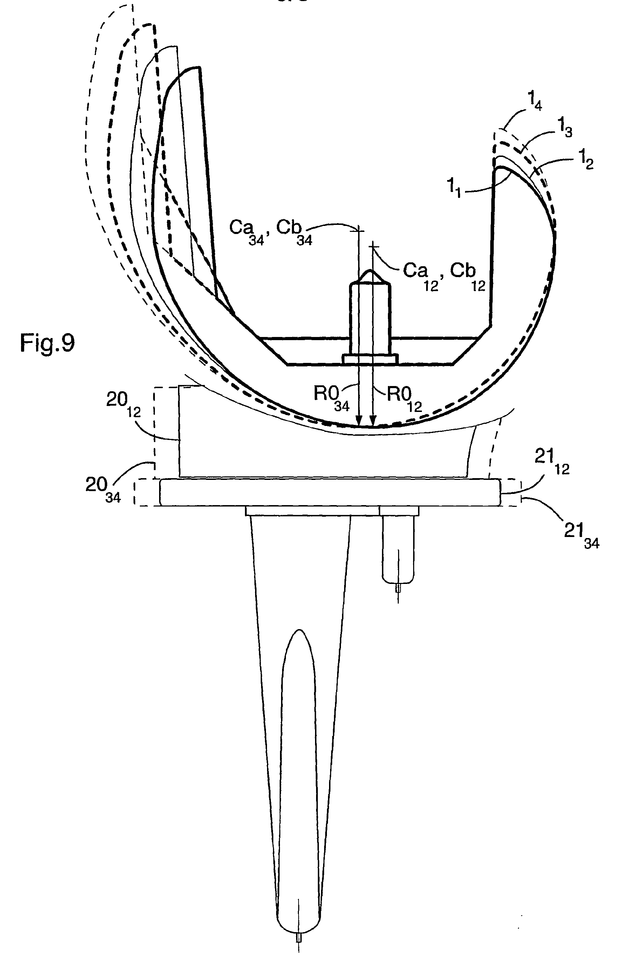

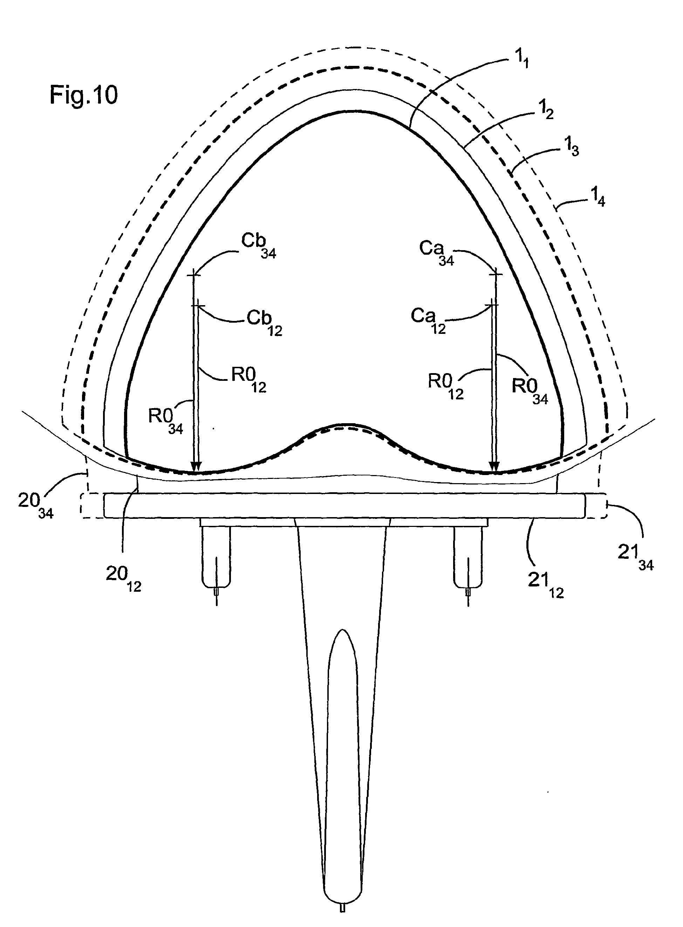

La prothèse de genou selon l'invention est prévue en différentes tailles, typiquement quatre à six tailles incrémentées en largeur antéro-postérieure et en largeur médio-latérale d'une valeur d'environ 4 mm. Les figures 9 et 10 montrent, à titre d'exemple, quatre implants fémoraux 1₁, 1₂, 1₃, 1₄ de tailles différentes. On notera, en référence également à la figure 7B, que l'orientation sagittale, β1, de chaque surface postérieure 8a, 8b, c'est-à-dire l'angle que fait, dans le plan sagittal, chaque surface postérieure 8a, 8b avec une direction de référence, par exemple la direction verticale, la longueur sagittale L2 et l'orientation sagittale β2 de chaque chanfrein postérieur 9a, 9b, la longueur sagittale L3 et l'orientation sagittale β3 de chaque surface distale 10a, 10b, l'orientation sagittale β4 du chanfrein postérieur 11, l'orientation sagittale β5 et l'origine sagittale PS sur le chanfrein antérieur 11 du chanfrein 18', la position sagittale des plots d'ancrage 15a, 15b, et la géométrie et les dimensions sagittales de la surépaisseur 16 sont constantes en fonction de la taille, et sont ainsi identiques pour tous les implants fémoraux 1₁ à 1₄. L'orientation sagittale β6 et la longueur sagittale L6 de la surface antérieure 12 peuvent être constantes ou varier en fonction de la taille.

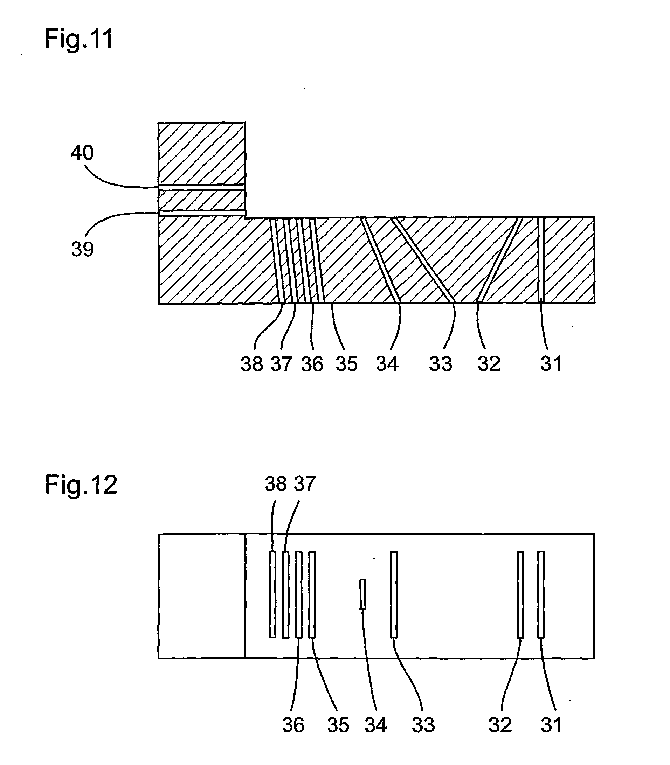

De la sorte, le nombre d'instruments de coupe nécessaires pour la préparation du fémur lors de l'intervention chirurgicale est réduit. Ainsi, par exemple, un seul instrument peut être prévu pour pratiquer, dans la partie distale du fémur, la coupe postérieure, le chanfrein postérieur, la coupe distale, le chanfrein antérieur et la coupe antérieure correspondant respectivement aux surfaces postérieures 8a, 8b, aux chanfreins postérieurs 9a, 9b, aux surfaces distales 10a, 10b, au chanfrein antérieur 11 et à la surface antérieure 12 de l'implant fémoral, ainsi que les coupes correspondant respectivement aux surépaisseurs 16 et 18. Les figures 11 et 12 montrent, à titre d'exemple, un tel instrument, comportant des fentes 31-34 pour, respectivement, la coupe postérieure, le chanfrein postérieur, le chanfrein antérieur et la coupe correspondant à la surépaisseur 18, des fentes 35-38 pour la coupe antérieure en fonction de la taille du fémur, et des fentes 39-40 pour la coupe distale et la coupe correspondant à la surépaisseur 16.

Toutefois, afin de mieux respecter l'anatomie naturelle du genou, réduire les risques de subluxation, et mieux épouser les formes de la partie distale du fémur, certaines caractéristiques de l'implant fémoral augmentent avec la taille, à savoir :
- le rayon R0 de la portion sphérique constituant la partie distale et postérieure inférieure de la surface articulaire 13a, 13b de chaque condyle,
- le rayon R1 du profil sagittal de la partie postérieure supérieure de la surface articulaire 13a, 13b de chaque condyle,
- la distance DT entre un plan coronal de référence PR tangent à la surface articulaire de chaque condyle lorsque la prothèse est en extension et le point le plus distal P1a, P1b de chaque condyle,
- la longueur sagittale L1 de chaque surface postérieure 8a, 8b,
- la longueur sagittale L5 du chanfrein 18',
- l'écartement (coronal) EC entre les centres de courbure respectifs Ca, Cb des portions sphériques des surfaces articulaires des condyles (la largeur de l'échancrure intercondylienne 3 ainsi que la géométrie et les dimensions dans le plan coronal de la surépaisseur 16 restent, en revanche, de préférence constantes), et
- l'écartement (coronal) entre les plots d'ancrage 15a, 15b.

La géométrie et les dimensions de l'insert tibial 20 sont bien entendu adaptées à celles de l'implant fémoral. Il a cependant été observé par le présent inventeur que l'on peut réduire le nombre d'inserts tibiaux nécessaires pour couvrir les différentes tailles de prothèse requises tout en conservant une bonne conformité à l'anatomie du genou, en faisant varier le rayon R0, la distance DT, l'écartement EC et l'écartement entre les plots d'ancrage 15a, 15b seulement toutes les deux tailles, comme représenté sur les figures 9 et 10. Dans l'exemple des figures 9 et 10, on peut voir que les deux plus petits implants fémoraux 1₁, 1₂ ont un même rayon de courbure R0₁₂ et des mêmes centres de courbure Ca₁₂, Cb₁₂, et les deux plus grands implants fémoraux 1₃, 1₄ ont un même rayon de courbure R0₃₄, supérieur au rayon R0₁₂, et des mêmes centres de courbure Ca₃₄, Cb₃₄. Ainsi, un même insert tibial 20₁₂ peut être utilisé pour les deux implants fémoraux les plus petits 1₁, 1₂, et un même insert tibial 20₃₄ peut être utilisé pour les deux implants fémoraux les plus grands 1₃, 1₄. Les rayons R0₁₂ et R0₃₄ sont égaux par exemple à 24 mm et 26 mm, respectivement. De préférence, la taille du plateau de l'embase tibiale varie également toutes les deux tailles, comme illustré aux figures 9 et 10 où deux tailles de plateau 21₁₂ et 21₃₄ sont représentées.

Par ailleurs, afin d'obtenir un même écartement en extension et en flexion entre les coupes respectives du fémur et du tibia lors de l'intervention chirurgicale, l'épaisseur, E1, de la partie distale 7a, 7b de chaque condyle est égale à l'épaisseur, E2, de la partie postérieure 6a, 6b de chaque condyle (figure 7B), ces deux épaisseurs E1, E2 restant constantes quelle que soit la taille de l'implant fémoral et ayant typiquement une valeur de 9,15 mm.

On observera, à la lumière des figures 9 et 10, qu'en augmentant le rayon R0 de la portion sphérique de la surface articulaire des condyles en fonction de la taille tout en maintenant constantes les épaisseurs distale et postérieure E1, E2 des condyles de l'implant fémoral, l'épaisseur de chaque condyle dans la zone du chanfrein postérieur 9a, 9b diminue, ce qui peut fragiliser l'implant fémoral au niveau des angles formés entre le chanfrein postérieur 9a, 9b, la surface postérieure 8a, 8b et la surface distale 10a, 10b. Dans la présente invention, lors de la conception de l'implant fémoral, l'on veille à ce que l'épaisseur de chaque condyle au niveau des angles précités reste au moins égale à une valeur minimale prédéterminée. Pour ce faire, l'on ne dessine le chanfrein postérieur 9a, 9b de chaque condyle qu'après avoir dessiné la surface articulaire 13a, 13b et les surfaces postérieure et distale de contact avec le fémur 8a, 8b, 10a, 10b de chaque condyle.

La présente invention a été exposée ci-dessous à titre d'exemple uniquement. Il apparaîtra clairement à l'homme du métier que des modifications peuvent être faites sans sortir du cadre de l'invention revendiquée. En particulier :
- les chanfreins postérieurs 9a, 9b pourraient être supprimés ; dans un tel cas, la surface de contact avec le fémur de l'implant fémoral ne comporterait que deux surfaces postérieures, deux surfaces distales, un chanfrein antérieur et une surface antérieure, et chaque surface postérieure formerait un angle (droit) avec la surface distale correspondante ;
- la surface antérieure 12 pourrait également être supprimée ; dans ce cas, le rebord antérieur 5 s'arrêterait au niveau de l'extrémité supérieure du chanfrein antérieur 11, et la prothèse selon l'invention serait une prothèse fémoro-tibiale, l'articulation fémoro-patellaire n'étant pas prothésée ;
- l'on pourrait ne prévoir qu'un seul condyle au lieu de deux ;
- la came pourrait se présenter sous une autre forme qu'une barre intercondylienne, à savoir par exemple sous la forme d'une surface d'un troisième condyle situé entre un condyle médial et un condyle latéral de l'implant fémoral ;
- l'insert tibial pourrait être fixe par rapport à l'embase tibiale, même si la solution dans laquelle l'insert tibial est mobile en rotation par rapport à l'embase tibiale apparaît préférable dans le cas où la prothèse est postéro-stabilisée; en variante, également, l'insert tibial pourrait être mobile à la fois en rotation et en translation par rapport à l'embase tibiale, de manière connue en soi ;
- le profil coronal de la partie distale et postérieure de la surface articulaire de chaque condyle pourrait être plat ou en arc de cercle de rayon différent de celui de la portion en arc de cercle du profil sagittal.

## Revendications

1. Système prothétique pour le genou comprenant un ensemble d'implants fémoraux (1₁ - 1₄) de tailles respectives croissantes, chacun desdits implants fémoraux comprenant au moins un condyle (4a, 4b) et un rebord antérieur (5), le ou chaque condyle comprenant des surfaces planes postérieure et distale (8a, 8b, 10a, 10b) de contact avec le fémur et une surface articulaire (13a, 13b), le rebord antérieur comprenant un chanfrein plan antérieur (11) de contact avec le fémur, l'orientation (β1) dans le plan sagittal de la ou chaque surface plane postérieure, l'orientation et la longueur (β3, L3) dans le plan sagittal de la ou chaque surface plane distale et l'orientation (β4) dans le plan sagittal du chanfrein plan antérieur étant identiques pour tous les implants fémoraux dudit ensemble, la longueur (L4) dans le plan sagittal du chanfrein plan antérieur augmentant avec la taille pour tous les implants fémoraux dudit ensemble, **caractérisé en ce que** le rayon de courbure (R0, R0₁₂, R0₃₄) dans le plan sagittal d'une partie distale de la surface articulaire du ou de chaque condyle augmente avec la taille pour au moins deux implants fémoraux (1₂, 1₃) dudit ensemble.

2. Système prothétique selon la revendication 1, **caractérisé en ce que** la distance (DT) entre un plan coronal de référence (PR) tangent à la surface articulaire (13a, 13b) du ou de chaque condyle lorsque le genou est en extension et le point le plus distal (P1a, P1b) du profil sagittal de la surface articulaire du ou de chaque condyle augmente avec la taille pour lesdits au moins deux implants fémoraux (1₂, 1₃) dudit ensemble.

3. Système prothétique selon la revendication 1 ou 2, **caractérisé en ce que** le rayon de courbure (R0, R0₁₂, R0₃₄) dans le plan coronal de la partie distale de la surface articulaire (13a, 13b) du ou de chaque condyle augmente avec la taille pour lesdits au moins deux implants fémoraux (1₂, 1₃) dudit ensemble.

4. Système prothétique selon l'une quelconque des revendications 1 à 3, **caractérisé en ce que** la longueur (L1) dans le plan sagittal de la surface plane postérieure (8a, 8b) de contact avec le fémur du ou de chaque condyle augmente avec la taille pour tous les implants fémoraux (1₁ - 1₄) dudit ensemble.

5. Système prothétique selon l'une quelconque des revendications 1 à 4, **caractérisé en ce que** le ou chaque condyle (4a, 4b) de chaque implant fémoral (1,1₁ - 1₄) comprend en outre, entre la surface plane postérieure (8a, 8b) et la surface plane distale (10, 10b), un chanfrein plan postérieur (9a, 9b) de contact avec le fémur, et **en ce que** l'orientation et la longueur (β2, L2) dans le plan sagittal de ce chanfrein plan postérieur sont identiques pour tous les implants fémoraux (1₁ - 1₄) dudit ensemble.

6. Système prothétique selon l'une quelconque des revendications 1 à 5, **caractérisé en ce que** le rebord antérieur (5) comprend en outre, entre le chanfrein plan antérieur (11) et une extrémité supérieure du rebord antérieur, une surface plane antérieure (12) de contact avec le fémur.

7. Système prothétique selon la revendication 6, **caractérisé en ce que** le rebord antérieur (5) de chaque implant fémoral (1₁ - 1₄) comprend en outre une surface articulaire (14) présentant une gorge (17) destinée à coopérer avec la rotule ou un implant rotulien, **en ce qu'**une surépaisseur (18) ayant un profil sagittal triangulaire est prévue sur la surface de contact avec le fémur du rebord antérieur (5) au niveau de l'angle formé par le chanfrein plan antérieur (11) et la surface plane antérieure (12) et dans une zone opposée à la gorge (17), cette surépaisseur (18) définissant un chanfrein (18') dont le profil sagittal a un point d'origine (PS) sur le chanfrein plan antérieur (11) et une orientation (β5) qui sont identiques pour tous les implants fémoraux (1₁ - 1₄) dudit ensemble et une longueur (L5) qui augmente avec la taille pour tous les implants fémoraux dudit ensemble.

8. Système prothétique selon l'une quelconque des revendications 1 à 7, **caractérisé en ce que** l'épaisseur (E1) d'une partie distale du ou de chaque condyle et l'épaisseur (E2) d'une partie postérieure du ou de chaque condyle sont identiques pour tous les implants fémoraux (1₁ - 1₄) dudit ensemble.

9. Système prothétique selon la revendication 8, **caractérisé en ce que** les épaisseurs respectives (E1, E2) de la partie distale et de la partie postérieure du ou de chaque condyle de chaque implant fémoral (1) sont sensiblement égales.

10. Système prothétique selon l'une quelconque des revendications 1 à 9, **caractérisé en ce que** la partie distale de la surface articulaire (13a, 13b) du ou de chaque condyle de chaque implant fémoral (1) est sensiblement une portion de sphère.

11. Système prothétique selon l'une quelconque des revendications 1 à 10, **caractérisé en ce que** chaque implant fémoral (1,1₁ - 1₄) comprend des premier et second condyles (4a, 4b).

12. Système prothétique selon la revendication 11 lorsqu'elle dépend de la revendication 10, **caractérisé en ce que** l'écartement (EC) entre les centres de courbure respectifs (Ca, Cb) des parties distales des surfaces articulaires (13a, 13b) des condyles (4a, 4b) augmente avec la taille pour lesdits au moins deux implants fémoraux (1₂, 1₃) dudit ensemble.

13. Système prothétique selon la revendication 12, **caractérisé en ce que** le rayon de courbure (R0, R0₁₂, R0₃₄) de la partie distale de la surface articulaire (13a, 13b) de chaque condyle et l'écartement (EC) entre les centres de courbure respectifs (Ca, Cb) des parties distales des surfaces articulaires (13a, 13b) des condyles (4a, 4b) sont identiques pour au moins deux implants fémoraux (1₁, 1₂) dudit ensemble.

14. Système prothétique selon la revendication 13, **caractérisé en ce que** le rayon de courbure (R0, R0₁₂, R0₃₄) de la partie distale de la surface articulaire (13a, 13b) de chaque condyle et l'écartement (EC) entre les centres de courbure respectifs (Ca, Cb) des parties distales des surfaces articulaires (13a, 13b) des condyles (4a, 4b) augmentent toutes les deux tailles d'implant fémoral.

15. Système prothétique selon l'une quelconque des revendications 1 à 14, **caractérisé en ce qu'**il comprend en outre au moins un insert tibial (20, 20₁₂, 20₃₄), le ou chaque insert tibial comprenant au moins une surface articulaire (240a, 240b) apte(s) à coopérer avec la ou les surfaces articulaires (13a, 13b) du ou des condyles d'au moins un implant fémoral correspondant (1,1₁ - 1₄) pour permettre une flexion du genou.

16. Système prothétique selon l'une quelconque des revendications 1 à 14, **caractérisé en ce qu'**il comprend en outre au moins un implant tibial (2, 20₁₂ - 21₁₂, 20₃₄ - 21₃₄), le ou chaque implant tibial comprenant au moins une surface articulaire (240a, 240b) apte(s) à coopérer avec la ou les surfaces articulaires (13a, 13b) du ou des condyles d'au moins un implant fémoral correspondant (1,1₁ - 1₄) pour permettre une flexion du genou.

17. Système prothétique selon la revendication 16 lorsqu'elle dépend de la revendication 10, **caractérisé en ce que** la ou chaque surface articulaire (240a, 240b) du ou de chaque implant tibial est sensiblement une portion de sphère de même rayon que le rayon (R0) de la partie distale de la surface articulaire (13a, 13b) du ou de chaque condyle du ou de chaque implant fémoral (1,1₁ - 1₄) correspondant.

18. Système prothétique selon la revendication 16 ou 17, **caractérisé en ce qu'**il comprend un ensemble d'implants tibiaux (20₁₂ - 21₁₂, 20₃₄ - 21₃₄) de tailles différentes.

19. Système prothétique selon l'une quelconque des revendications 16 à 18, **caractérisé en ce que** chaque implant fémoral comprend une came (26) et le ou chaque implant tibial comprend un plot (25) apte à coopérer avec la came d'au moins un implant fémoral correspondant à partir d'un certain angle de flexion du genou pour stabiliser postérieurement cet implant fémoral par rapport à l'implant tibial, **en ce que** la surface articulaire (13a, 13b) du ou de chaque condyle de chaque implant fémoral est congruente dans le plan sagittal avec la surface articulaire (240a, 240b) correspondante de l'implant tibial correspondant pour une flexion du genou allant au moins jusqu'à un angle déterminé (α), et **en ce que** le plot (25) du ou de chaque implant tibial et la came (26) du ou de chaque implant fémoral correspondant sont agencés pour que la came entre en contact avec le plot à un angle de flexion du genou sensiblement égal à l'angle déterminé (α).

20. Système prothétique selon la revendication 19, **caractérisé en ce que** l'angle déterminé (α) est sensiblement égal à 90°.

## Claims

1. Prosthetic system for the knee, including a set of femoral implants (1₁ - 14) of increasing respective sizes, each of the said femoral implants including at least one condyle (4a, 4b) and an anterior flange (5), the condyle or each condyle including posterior and distal flat surfaces (8a, 8b, 10a, 10b) for contact with the femur and an articular surface (13a, 13b), the anterior flange including an anterior flat chamfer (11) for contact with the femur, the orientation (β1) of the one or each posterior flat surface in the sagittal plane, the orientation and the length (β3, L3) of the one or each distal flat surface in the sagittal plane and the orientation (β4) of the anterior flat chamfer in the sagittal plane being identical for all the femoral implants of the said set, the length (L4) of the anterior flat chamfer in the sagittal plane increasing with size for all the femoral implants of the said set, **characterised in that** the radius of curvature (R0, R0₁₂, R0₃₄) of a distal part of the articular surface of the one or each condyle in the sagittal plane increases with size for at least two femoral implants (1₂, 1₃) of the said set.

2. Prosthetic system according to Claim 1, **characterised in that** the distance (DT) between a reference coronal plane (PR) tangent to the articular surface (13a, 13b) of the one or each condyle when the knee is extended and the most distal point (P1a, P1b) of the sagittal profile of the articular surface of the one or each condyle increases with size for at least two femoral implants (1₂, 1₃) of the said set.

3. Prosthetic system according to Claim 1 or 2, **characterised in that** the radius of curvature (R0, R0₁₂, R0₃₄) of the distal part of the articular surface (13a, 13b) of the one or each condyle in the coronal plane increases with size for the said at least two femoral implants (1₂, 1₃) of the said set.

4. Prosthetic system according to any of Claims 1 to 3, **characterised in that** the length (L1) in the sagittal plane of the posterior flat surface (8a, 8c) for contact with the femur of the one or each condyle increases with size for all the femoral implants (1₂, 1₃) of the said set.

5. Prosthetic system according to any of Claims 1 to 4, **characterised in that** the one or each condyle (4a, 4b) of each femoral implant (1, 1₁ - 1₄) additionally includes a posterior flat chamfer (9a, 9b) for contact with the femur between the posterior flat surface (8a, 8b) and the distal flat surface (10, 10b), and **in that** the orientation and the length (β2, L2) of this posterior flat chamfer in the sagittal plane are identical for all the femoral implants (1₁ - 1₄) of the said set.

6. Prosthetic system according to any of Claims 1 to 5, **characterised in that** the anterior flange (5) additionally includes an anterior flat surface (12) for contact with the femur between the anterior flat chamfer (11) and an upper end of the anterior flange.

7. Prosthetic system according to Claim 6, **characterised in that** the anterior flange (5) of each femoral implant (1₁ - 1₄) additionally includes an articular surface (14) presenting a groove (17) intended to cooperate with the patella or a patella implant, **in that** a thickening (18) having a triangular sagittal profile is provided on the femoral contact surface of the anterior flange (5) at the level of the angle formed by the anterior flat chamfer (11) and the anterior flat surface (12) and in a zone opposite the groove (17), this thickening (18) defining a chamfer (18') whose sagittal profile has a point of origin (PS) on the anterior flat chamfer (11) and an orientation (β5) which are identical for all the femoral implants (1₁ - 1₄) of the said set and a length (L5) which increases with size for all the femoral implants of the said set.

8. Prosthetic system according to any of Claims 1 to 7, **characterised in that** the thickness (E1) of a distal part of the one of each condyle and the thickness (E2) of the posterior part of the one or each condyle are identical for all the femoral implants (1₁ - 14) of the said set.

9. Prosthetic system according to Claim 8, **characterised in that** the respective thicknesses (E1, E2) of the distal part and of the posterior part of the one or each condyle of each femoral implant (1) are substantially equal.

10. Prosthetic system according to any of Claims 1 to 9, **characterised in that** the distal part of the articular surface (13a, 13b) of the one or each condyle of each femoral implant (1) is substantially a portion of a sphere.

11. Prosthetic system according to any of Claims 1 to 10, **characterised in that** each femoral implant (1, 1₁ - 1₄) includes first and second condyles (4a, 4b).

12. Prosthetic system according to Claim 11 when it depends on Claim 10, **characterised in that** the distance (EC) between the respective centres of curvature (Ca, Cb) of the distal parts of the articular surfaces (13a, 13b) of the condyles (4a, 4b) increases with size for the said at least two femoral implants (1₂, 1₃) of the said set.

13. Prosthetic system according to Claim 12, **characterised in that** the radius of curvature (R0, R0₁₂, R0₃₄) of the distal part of the articular surface (13a, 13b) of each condyle and the distance (EC) between the respective centres of curvature (Ca, Cb) of the distal parts of the articular surfaces (13a, 13b) of the condyles (4a, 4b) are identical for at least two femoral implants (1₁, 1₂) of the said set.

14. Prosthetic system according to Claim 13, **characterised in that** the radius of curvature (R0, R0₁₂, R0₃₄) of the distal part of the articular surface (13a, 13b) of each condyle and the distance (EC) between the respective centres of curvature (Ca, Cb) of the distal parts of the articular surfaces (13a, 13b) of the condyles (4a, 4b) increase every second femoral implant size.

15. Prosthetic system according to any of Claims 1 to 14, **characterised in that** it additionally includes at least one tibial insert (20, 20₁₂, 20₃₄), whereby the one or each tibial insert includes at least one articular surface (240a, 240b) able to cooperate with the articular surface or surfaces (13a, 13b) of the one or each condyle of at least one corresponding femoral implant (1, 1₁ - 1₄) to allow flexion of the knee.

16. Prosthetic system according to any of Claims 1 to 14, **characterised in that** it additionally includes at least one tibial insert (2, 20₁₂ - 21₁₂, 20₃₄ - 21₃₄), whereby the one or each tibial insert includes at least one articular surface (240a, 240b) able to cooperate with the articular surface or surfaces (13a, 13b) of the one or each condyle of at least one corresponding femoral implant (1, 1₁ - 1₄) to allow flexion of the knee.

17. Prosthetic system according to Claim 16 when it depends on Claim 10, **characterised in that** the one or each articular surface (240a, 240b) of the one or each tibial implant is substantially a portion of a sphere of the same radius as the radius (R0) of the distal part of the articular surface (13a, 13b) of the one or each condyle of the one or each corresponding femoral implant (1, 1₁ - 1₄).

18. Prosthetic system according to Claims 16 or 17, **characterised in that** it includes a set of tibial implants (20₁₂ - 21₁₂, 20₃₄ - 21₃₄) of different sizes.

19. Prosthetic system according to any of Claims 16-18, **characterised in that** each femoral implant includes a cam (26) and that the one or each tibial implant includes a stud (25) able to cooperate with the cam of at least one corresponding femoral implant from a certain angle of knee flexion in order to provide posterior stabilisation of this femoral implant with regard to the tibial implant, and **in that** the articular surface (13a, 13b) of the one or each condyle of each femoral implant is congruent in the sagittal plane with the corresponding articular surface (240a, 240b) of the corresponding tibial implant for knee flexion going at least up to a determined angle (α), and **in that** the stud (25) of the one or each tibial implant and the cam (26) of the one or each corresponding femoral implant are arranged so that the cam comes into contact with the stud at an angle of knee flexion substantially equal to the determined angle (α).

20. Prosthetic system according to Claim 19, **characterised in that** the determined angle (α) is substantially equal to 90°.

## Patentansprüche

1. Prothesesystem für das Knie, enthaltend einen Satz Femurimplantate (1₁ - 1₄) in jeweilig zunehmenden Grössen, wobei jedes der genannten Femurimplantate mindestens eine Kondyle (4a, 4b) und einen vorderen Rand (5) umfasst, wobei die oder jede Kondyle hintere und distale ebene Flächen (8a, 8b, 10a, 10b) für den Kontakt mit dem Femur und eine Gelenkfläche (13a, 13b) umfasst, und der vordere Rand eine vordere ebene Abschrägung (11) für den Kontakt mit dem Femur umfasst, wobei die Ausrichtung (β1) in der Sagittalebene der oder jeder hinteren ebenen Fläche, die Ausrichtung und die Länge (β3, L3) in der Sagittalebene der oder jeder ebenen Distalfläche und die Ausrichtung (β4) in der Sagittalebene der vorderen ebenen Abschrägung für alle Femurimplantate des besagten Implantatsatzes identisch sind, wobei die Länge (L4) in der Sagittalebene der vorderen ebenen Abschrägung mit der Grösse für alle Femurimplantate des besagten Implantatsatzes ansteigt, **gekennzeichnet dadurch, dass** der Krümmungsradius (R0, R0₁₂, R0₃₄) in der Sagittalebene eines Distalteiles der Gelenkfläche der oder jeder Kondyle mit der Grösse für mindestens zwei Femurimplantate (1₂, 1₃) des besagten Implantatsatzes ansteigt.

2. Prothesesystem nach Anspruch 1, **gekennzeichnet dadurch, dass** der Abstand (DT) zwischen einer Koronarreferenzebene (PR), die der Gelenkfläche (13a, 13b) der oder jeder Kondyle anliegt, wenn das Knie gedehnt ist und der distalste Punkt (P1a, P1b) des Sagittalprofils der Gelenkfläche der oder jeder Kondyle mit der Grösse für die besagten mindestens zwei Femurimplantate (1₂, 1₃) des besagten Implantatsatzes ansteigt.

3. Prothesesystem nach Anspruch 1 oder 2, **gekennzeichnet dadurch, dass** der Krümmungsradius (R0, R0₁₂, R0₃₄) in der Koronarebene des Distalteils der Gelenkfläche (13a, 13b) der oder jeder Kondyle mit der Grösse für die besagten mindestens zwei Femurimplantate (1₂, 1₃) des besagten Implantatsatzes ansteigt.

4. Prothesesystem nach irgendeinem der Ansprüche 1 bis 3, **gekennzeichnet dadurch, dass** die Länge (L1) in der Sagittalebene der hinteren ebenen Fläche (8a, 8b) für den Kontakt mit dem Femur der oder jeder Kondyle mit der Grösse für alle Femurimplantate (1₁ - 1₄) des besagten Implantatsatzes ansteigt.

5. Prothesesystem nach irgendeinem der Ansprüche 1 bis 4, **gekennzeichnet dadurch, dass** die oder jede Kondyle (4a, 4b) eines jeden Femurimplantates (1,1₁ -1₄) ferner zwischen der hinteren ebenen Fläche (8a, 8b) und der ebenen Distalfläche (10,10b) eine hintere ebene Abschrägung (9a, 9b) für den Kontakt mit dem Femur umfasst, und **gekennzeichnet dadurch, dass** die Ausrichtung und die Länge (β2, L2) in der Sagittalebene dieser hinteren ebenen Abschrägung für alle Femurimplantate (1₁ - 1₄) des besagten Implantatsatzes identisch sind.

6. Prothesesystem nach irgendeinem der Ansprüche 1 bis 5, **gekennzeichnet dadurch, dass** der vordere Rand (5) ferner zwischen der vorderen ebenen Abschrägung (11) und einem oberen Ende des vorderen Randes eine vordere ebene Fläche (12) für den Kontakt mit dem Femur umfasst.

7. Prothesesystem nach Anspruch 6, **gekennzeichnet dadurch, dass** der vordere Rand (5) eines jeden Femurimplantates (1₁ - 1₄) ferner eine Gelenkfläche (14) umfasst, die eine Kehle (17) beinhaltet, der dazu dient, mit der Kniescheibe oder einem Implantat der Kniescheibe zusammenzuwirken, **dadurch**, dass eine Überhöhung (18), enthaltend ein dreieckiges Sagittalprofil, auf der Kontaktfläche mit dem Femur des vorderen Randes (5) am Winkel, der durch die vordere ebene Abschrägung (11) und die vordere ebene Fläche (12) gebildet wird, und im gegenüberliegenden Bereich der Kehle (17) vorgesehen ist, wobei diese Überhöhung (18) eine Abschrägung (18') ausbildet, deren Sagittalprofil einen Anfangspunkt (PS) an der vorderen ebenen Abschrägung (11) und eine Ausrichtung (β5) hat, welche für alle Femurimplantaten (1₁ - 1₄) des besagten Implantatsatzes identisch sind sowie eine Länge (L5), die mit der Grösse für alle Femurimplantate des besagten Implantatsatzes ansteigt.

8. Prothesesystem nach irgendeinem der Ansprüche 1 bis 7, **gekennzeichnet dadurch, dass** die Dicke (E1) eines Distalteiles der oder jeder Kondyle und die Dicke (E2) eines hinteren Teiles einer oder jeder Kondyle für alle Femurimplantate (1₁ - 1₄) des besagten Implantatsatzes identisch sind.

9. Prothesesystem nach Anspruch 8, **gekennzeichnet dadurch, dass** die jeweilige Dicke (E1, E2) des Distalteiles und des hinteren Teiles der oder jeder Kondyle jedes Femurimplantates (1) merklich gleich sind.

10. Prothesesystem nach irgendeinem der Ansprüche 1 bis 9, **gekennzeichnet dadurch, dass** das Distalteil der Gelenkfläche (13a, 13b) der oder jeder Kondyle jedes Femurimplantates (1) merklich ein Teil einer Kugel ist.

11. Prothesesystem nach irgendeinem der Ansprüche 1 bis 10, **gekennzeichnet dadurch, dass** jedes Femurimplantat (1, 1₁ - 1₄) erste und zweite Kondylen (4a, 4b) umfasst.

12. Prothesesystem nach Anspruch 11, wenn dieser von Anspruch 10 abhängt, **gekennzeichnet dadurch, dass** der Abstand (EC) zwischen den jeweiligen Krümmungsmittelpunkten (Ca, Cb) der Distalteile der Gelenkflächen (13a, 13b) der Kondylen (4a, 4b) mit der Grösse für die besagten mindestens zwei Femurimplantate (1₂, 1₃) des besagten Implantatsatzes ansteigt.

13. Prothesesystem nach Anspruch 12, **gekennzeichnet dadurch, dass** der Krümmungsradius (R0, R0₁₂, R0₃₄) des Distalteils der Gelenkfläche (13a, 13b) jeder Kondyle und der Abstand (EC) zwischen den jeweiligen Krümmungsmittelpunkten (Ca, Cb) der Distalteile der Gelenkflächen (13a, 13b) der Kondylen (4a, 4b) für mindestens zwei Femurimplantaten (1₂, 1₃) des besagten Implantatsatzes identisch sind.

14. Prothesesystem nach Anspruch 13, **gekennzeichnet dadurch, dass** der Krümmungsradius (R0, R0₁₂, R0₃₄) des Distalteiles der Gelenkfläche (13a, 13b) jeder Kondyle und der Abstand (EC) zwischen den jeweiligen Krümmungsmittelpunkten (Ca, Cb) der Distalteile der Gelenkflächen (13a, 13b) der Kondylen (4a, 4b) alle zwei Grössen des Femurimplantates ansteigen.

15. Prothesesystem nach irgendeinem der Ansprüche 1 bis 14, **gekennzeichnet dadurch, dass** es ferner mindestens ein tibiales Inlay (2, 20₁₂ - 21₁₂, 20₃₄ - 21₃₄) umfasst, wobei das oder jedes tibiale Inlay mindestens eine Gelenkfläche (240a, 240b) umfasst, die mit der oder den Gelenkflächen (13a, 13b) der öder den Kondyle(n) von mindestens einem entsprechenden Femurimplantat (1,1₁-1₄) zusammenwirken kann, um eine Beugung des Knies zu erlauben.

16. Prothesesystem nach irgendeinem der Ansprüche 1 bis 14, **gekennzeichnet dadurch, dass** es ferner mindestens ein Tibiaimplantat enthält (2, 20₁₂ - 21₁₂, 20₃₄ - 21₃₄), wobei das oder jedes Tibiaimplantat mindestens eine Gelenkfläche (240a, 240b) umfasst, die mit der oder den Gelenkflächen (13a, 13b) der Kondyle(n) von mindestens einem entsprechenden Femurimplantat (1, 1₁ - 1₄) zusammenwirken kann, um eine Beugung des Knies zu erlauben.

17. Prothesesystem nach Anspruch 16, wenn dieser von Anspruch 10 abhängt, **gekennzeichnet dadurch, dass** die oder jede Gelenkfläche (240a, 240b) des oder jedes Tibiaimplantates merklich ein Teil einer Kugel ist mit dem gleichen Radius wie der Radius (R0) des Distalteiles der Gelenkfläche (13a, 13b) der oder jeder Kondyle des oder jedes entsprechenden Femurimplantates (1,1₁-1₄).

18. Prothesesystem nach Anspruch 16 oder 17, **gekennzeichnet dadurch, dass** es einen Satz Tibiaimplantate (2, 20₁₂ - 21₁₂,20₃₄ - 21₃₄) verschiedener Grössen enthält.

19. Prothesesystem nach irgendeinem der Ansprüche 16 bis 18, **gekennzeichnet dadurch, dass** jedes Femurimplantat eine Nocke (26) enthält und dass das oder jedes Tibiaimplantat einen Kontaktteil (25) umfasst, der geeignet ist, mit der Nocke von mindestens einem entsprechenden Femurimplantat, ausgehend von einem bestimmten Beugungswinkel des Knies, zusammenzuwirken, um dieses Femurimplantat im Verhältnis zum Tibiaimplantat hinten zu stabilisieren, **dadurch gekennzeichnet, dass** die Gelenkfläche (13a, 13b) der oder jeder Kondyle jedes Femurimplantates in der Sagittalebene mit der entsprechenden Gelenkfläche (240a, 240b) des entsprechenden Tibiaimplantates bei einer Beugung des Knies kongruent ist, wobei die Beugung mindestens bis zu einem bestimmten Winkel (α) geht, und **gekennzeichnet dadurch, dass** der Kontaktteil (25) des oder jedes Tibiaimplantates und die Nocke (26) des oder jedes entsprechenden Femurimplantates so eingerichtet sind, dass die Nocke bei einem Beugungswinkel des Knies in Kontakt mit dem Kontaktteil tritt, der merklich dem bestimmten Winkel (α) entspricht.

20. Prothesesystem nach Anspruch 19, **gekennzeichnet dadurch, dass** der bestimmte Winkel (α) merklich 90° beträgt.
